(19) Europäisches Patentamt / European Patent Office / Office européen des brevets

(11) **EP 2 336 158 B1**

(12) **EUROPEAN PATENT SPECIFICATION**

(45) Date of publication and mention
of the grant of the patent:
**14.08.2013 Bulletin 2013/33**

(51) Int Cl.:
*C07K 14/47* (2006.01)     *G01N 33/58* (2006.01)
*G01N 33/563* (2006.01)

(21) Application number: **10195875.9**

(22) Date of filing: **20.12.2010**

(54) **Dry analytical element for measurement of canine CRP**

Trockenes Analyseelement zur Messung von CRP bei Hunden

Élément analytique sec pour la mesure de la protéine C réactive canine

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2009 JP 2009289288**

(43) Date of publication of application:
**22.06.2011 Bulletin 2011/25**

(83) **Declaration under Rule 32(1) EPC (expert
solution)**

(73) Proprietor: **FUJIFILM Corporation
Minato-ku
Tokyo 106-8620 (JP)**

(72) Inventors:
• **Toda, Satoru
Kanagawa 258-8577 (JP)**
• **Nakamura, Kentaro
Kanagawa 258-8577 (JP)**

(74) Representative: **HOFFMANN EITLE
Patent- und Rechtsanwälte
Arabellastrasse 4
81925 München (DE)**

(56) References cited:
EP-B1- 0 431 171     JP-A- 53 086 015
JP-A- 61 243 363     JP-A- 62 218 866
US-A- 5 500 345     US-A1- 2006 029 976

• MADS KJELGAARD-HANSEN ET AL: "Evaluation of a commercially available human C-reactive protein (CRP) turbidometric immunoassay for determination of canine serum CRP concentration", 1 January 2003 (2003-01-01), VETERINARY CLINICAL PATHOLOGY, VETERINARY PRACTICE PUBLISHING, MISSION VIEJO, CA, US, PAGE(S) 81 - 87, XP002546868, ISSN: 0275-6282 * abstract *
• KJELGAARD-HANSEN M ET AL: "Canine serum C-reactive protein detected by means of a near-patient test for human C-reactive protein", 1 June 2008 (2008-06-01), JOURNAL OF SMALL ANIMAL PRACTICE, BLACKWELL SCIENTIFIC PUBLICATIONS, OXFORD, GB, PAGE(S) 282 - 286, XP002546870, ISSN: 0022-4510 * the whole document *
• FRANSSON BOEL A ET AL: "Assessment of three automated assays for C-reactive protein determination in dogs", 1 December 2007 (2007-12-01), AMERICAN JOURNAL OF VETERINARY RESEARCH, AMERICAN VETERINARY MEDICINE ASSOCIATION, US, PAGE(S) 1281 - 1286, XP009123080, ISSN: 0002-9645 * the whole document *
• KJELGAARD-HANSEN M ET AL: "Evaluation of a commercially available enzyme-linked immunosorbent assay (ELISA) for the determination of C-reactive protein in canine serum", 11 April 2003 (2003-04-11), JOURNAL OF VETERINARY MEDICINE. SERIES A - ZENTRALBLATT FUERVETERINAERMEDIZIN. REIHE A, PAREY, BERLIN, DE, PAGE(S) 164 - 168, XP009144802, ISSN: 0931-184X * the whole document *

Remarks:
The file contains technical information submitted after the application was filed and not included in this specification

Remarks:
The file contains technical information submitted after the application was filed and not included in this specification

**Description**

Technical Field

The present invention relates to a dry analytical element for the measurement of canine CRP, a reagent or a kit for the measurement of canine CRP, and a method for measuring canine CRP, wherein a monoclonal antibody that recognizes the canine CRP is used.

Background Art

C-reactive protein (CRP) has been known as a protein whose production amount correlates with the strength of an inflammatory response. Thus, a value obtained by measuring the amount of CRP in serum can be used as an indicator of an inflammatory response. That is to say, if the serum CRP value is high, it shows strong inflammation.

JP Patent Publication (Kokai) No. 62-210984 A (1987) describes that an anti-canine CRP monoclonal antibody is produced by a conventionally known production technique of monoclonal antibody and that an antigen (canine CRP) is then purified by a known ability chromatography technique using the obtained antibody. The anti-canine CRP monoclonal antibody described in JP Patent Publication (Kokai) No. 62-210984 A (1987) is characterized in that it captures canine CRP, and JP Patent Publication (Kokai) No. 62-210984 A (1987) does not describe any assay of canine CRP.

Vet. clin. Pathol. 2003, 32: 81-87 describes that an antibody used in a test kit of immunonephelometry, which is used for the measurement of human CRP, has reactivity with canine CRP. The antibody described in Vet clin. Pathol. 2003, 32: 81-87 was not intentionally prepared in view of the reactivity with canine CRP, and this document does not describe a monoclonal antibody. Also, Vet. clin. Pathol. 2003, 32: 81-87 does not describe, either, a method for stably producing this antibody.

Japanese Patent No. 3151080 describes a dry immunoanalytical element for the measurement of human CRP, but does not describe the reactivity with canine CRP. Even if a canine serum is measured by using the dry immunoanalytical element for the measurement of human CRP described in Japanese Patent No. 3151080, results showing quantitative property are not obtained, and thus, this element cannot be used for the assay of canine CRP. This is because the specificity of the antibody used in Japanese Patent No. 3151080 is limited to human CRP and the antibody does not substantially recognize canine CRP.

JP Patent Publication (Kokai) No. 62-155299 A (1987) describes a reagent for simply detecting canine CRP, in which latex particles are sensitized with an anti-canine CRP antibody and the obtained antibody-sensitized particles are mixed into a buffer, and a method for simply detecting canine CRP using the above-mentioned reagent. The method described in JP Patent Publication (Kokai) No. 62-155299 A (1987) is highly sensitive, and it is a latex agglutination method in which the collected blood can be directly used as an analytical sample without any treatments. However, this method has been disadvantageous in terms of poor quantitative property.

JP Patent Publication No. 61-243363 A (1986) describes a CRP measurement method involving the use of an anti-CRP rabbit IgG and an anti-CRP goat IgG.

Summary of the Invention

It is an object of the present invention to solve the aforementioned problems of the prior art techniques. That is to say, it is an object of the present invention to provide a dry analytical element for the measurement of canine CRP, an immunoanalytical reagent or kit for the measurement of canine CRP, and a method for measuring canine CRP, wherein canine CRP can be accurately quantified at a good signal/noise ratio (S/N ratio).

The present inventors have conducted intensive studies directed towards achieving the aforementioned object. The inventors have first performed immunization of animals such as mice or rats, and have confirmed an increase in the antibody titer of serum with respect to the immunogen. Thereafter, the inventors have collected lymph cells or splenic cells, and they have then fused such cells with myeloma cells to produce hybridomas. Thereafter, the inventors have cultured the fused cells in a selective medium. When screening was performed using a culture supernatant of the proliferating hybridomas, the inventors have applied a method of sensitively reflecting the coupling constant of an antibody, in addition to a conventional qualitative screening method. As a result, the present inventors have succeeded in reliably selecting a hybridoma that produces a useful antibody. The thus obtained, canine CRP antibody producing hybridoma-derived antibody had a high affinity for an antigen. Further, with regard to the above-obtained canine CRP antibody, the present inventors have found that a dry analytical element for the measurement of canine CRP, which is capable of accurately quantifying canine CRP at a good signal/noise ratio (S/N ratio), can be provided by producing a dry analytical element according to the method described in Japanese Patent No. 3151080 with the combined use of two types of monoclonal antibodies, which satisfy all of the characteristics that a first monoclonal antibody and a second monoclonal antibody have a coupling constant of $10^7$ M$^{-1}$ to $10^9$ M$^{-1}$ with respect to canine CRP, the first monoclonal antibody and the second monoclonal antibody are of the IgG1 subclass, and the molar ratio between the first monoclonal antibody and the second monoclonal antibody is 1 to 20. The present invention has been completed based on these findings.

The present invention provides a dry analytical element for the measurement of canine CRP, which comprises, on a light-permeable support, an immune reaction layer comprising: a monoclonal antibody fragment prepared by labeling, with an enzyme, a first monoclonal antibody fragment that recognizes canine CRP; and a second monoclonal antibody that recognizes canine CRP, wherein the first monoclonal antibody and the second monoclonal antibody have a coupling constant of $10^7$ M$^{-1}$ to $10^9$ M$^{-1}$ with respect to canine CRP, the first monoclonal antibody and the second monoclonal antibody are of the IgG1 subclass, and the molar ratio between the first monoclonal antibody and the second monoclonal antibody is 1 to 20.

Preferably, the enzyme is *Bacillus subtilis* α-amylase.

Preferably, the dry analytical element for the measurement of canine CRP according to the present invention further comprises an amylase inhibitor for inhibiting canine amylase.

Preferably, the dry analytical element for the measurement of canine CRP according to the present invention comprises, on a light-permeable support,

(a) a detection layer for detecting an enzyme reaction product dispersed and moved from an immune reaction layer;
(b) a substrate layer comprising a substrate of the enzyme of the (c) below; and
(c) an immune reaction layer comprising: a monoclonal antibody fragment prepared by labeling, with an enzyme, a first monoclonal antibody fragment that recognizes canine CRP; and a second monoclonal antibody that recognizes canine CRP; in this order from the light-permeable support side.

Further preferably, the detection layer is a reagent layer which contains a reagent composition for detecting an enzyme reaction product (a diffusible substance) dispersed and moved from the immune reaction layer.

The present invention further provides an immunoanalytical reagent or kit for the measurement of canine CRP, which comprises: a monoclonal antibody fragment prepared by labeling, with an enzyme, a first monoclonal antibody fragment that recognizes canine CRP; and a second monoclonal antibody that recognizes canine CRP, wherein the first monoclonal antibody and the second monoclonal antibody have a coupling constant of $10^7$ M$^{-1}$ to $10^9$ M$^{-1}$ with respect to canine CRP, the first monoclonal antibody and the second monoclonal antibody are of the IgGI subclass, and the molar ratio between the first monoclonal antibody and the second monoclonal antibody is 1 to 20.

Preferably, the enzyme is *Bacillus subtilis* α-amylase.

Preferably, the immunoanalytical reagent or kit for the measurement of canine CRP according to the present invention further comprises an amylase inhibitor for inhibiting canine amylase.

The present invention further provides a method for measuring canine CRP, wherein the dry analytical element for the measurement of canine CRP of the present invention or the inmunoanalytical reagent or kit for the measurement of canine CRP of the present invention is used.

According to the dry analytical element for the measurement of canine CRP, mimunoanalytical reagent or kit for the measurement of canine CRP, and method for measuring canine CRP of the present invention, canine CRP can be accurately quantified at a good signal/noise ratio (S/N ratio).

Brief Description of the Drawings

Figure 1 shows the results obtained by measuring CRP in a canine serum, using the dry analytical element of the present invention.
Figure 2 shows the results obtained by measuring CRP in a canine serum, using the dry analytical element of the present invention.

Description of Embodiments

Hereinafter, the present invention will be more specifically described.

In the present invention, there are used a monoclonal antibody fragment prepared by labeling, with an enzyme, a first monoclonal antibody fragment that recognizes canine CRP, and a second monoclonal antibody that recognizes canine CRP. The above two types of antibodies used in the present invention satisfy the conditions that the first monoclonal antibody and the second monoclonal antibody have a coupling constant of $10^7$ M$^{-1}$ to $10^9$ M$^{-1}$ with respect to canine CRP, the first monoclonal antibody and the second monoclonal antibody are of the IgGI subclass, and the molar ratio between the first monoclonal antibody and the second monoclonal antibody is 1 to 20. It is to be noted that the term "antibody" is used in the present invention to mean not only an antibody molecule as a whole, but also a fragment thereof (for examples, Fab, F(ab')₂, and Fab' fragments).

According to one embodiment of the present invention, the monoclonal antibody fragment prepared by labeling, with an enzyme, the first monoclonal antibody fragment that recognizes canine CRP, and the second monoclonal antibody that recognizes canine CRP, both of which are used in the present invention, can be used in a dry analytical element for the measurement of canine CRP. In this case, the aforementioned dry analytical element for the measurement of canine CRP has a structure comprising an immune reaction layer, and the aforementioned antibodies are contained in this immune reaction layer. According to another embodiment of the present invention, an immunoanalytical reagent or kit for the measurement of canine CRP can be configured by combining the monoclonal antibody fragment prepared by labeling, with an enzyme, the first monoclonal antibody fragment that recognizes canine CRP, with the second monoclonal antibody that recognizes canine CRP, both of which are used in the present invention. Specifically, the monoclonal antibody fragment prepared by labeling, with an enzyme, the first monoclonal antibody fragment that recognizes canine CRP, may be mixed with the second monoclonal antibody that recognizes canine CRP, so as to prepare an immunoanalytical reagent for the measurement of canine CRP. Alternatively, the monoclonal antibody fragment prepared by labeling, with an enzyme, the first monoclonal antibody fragment that recognizes canine CRP, and the second monoclonal antibody that recognizes canine CRP, may be prepared, separately, and the two types of antibodies may be then combined, so as to prepare an immunoanalytical kit for the measurement of canine CARP.

Such a monoclonal antibody can be obtained by an ordinary method. Specifically, an antigen is injected into the abdominal cavity or the like several times together with an adjuvant, and splenic cells are then collected. Thereafter, using polyethylene glycol or the like, the collected cells are fused with mouse myeloma cells. Thereafter, antibody-producing cells are cloned from the fused cells, and they are then allowed to proliferate as monoclone cells. The thus proliferating cells are further injected into the abdominal cavity of a mouse to obtain ascites and serum that contain a monoclonal antibody. More specifically, a monoclonal antibody can be obtained as follows.

First CRP (canine CRP, etc.) is used as an antigen, and it is administered to a mammal such as a xat, a mouse, or a rabbit. The dose of the antigen per animal is 0.1 to 100 mg without the use of an adjuvant. It is 1 to 2000 μg with the use of an adjuvant. Examples of such an adjuvant include a Freund's complete adjuvant (FCA), a Freund's incomplete adjuvant (FIA), and an aluminum hydroxide adjuvant. Immunization is mainly carried out by injecting an antigen into the vein, subcutis, or abdominal cavity. The immunization interval is not particularly limited. Immunization is carried out at intervals of several days to several weeks, and preferably 2 to 5 weeks, 1 to 10 times, and preferably 2 to 5 times. Thereafter, antibody-producing cells are collected 1 to 60 days, and preferably 1 to 14 days after the final immunization. Such antibody-producing cells include splenic cells, lymph node cells, peripheral blood cells, and others. Splenic cells or regional lymph node cells are preferable.

To obtain cell fusion hybridomas, cell fusion is carried out by fusing antibody-producing cells with myeloma cells. As myeloma cells to be fused with antibody-producing cells, generally available cell lines established from animals such as mice can be used. The cell lines are preferably those, which do not survive in a HAT selective medium (containing hypoxanthine, aminopterm, and thymidine) in the state of unfused cells, and which survive only in a state in which they are fused with antibody-producing cells. Myeloma cells include mouse myeloma cell lines such as P3X63-Ag.8.U1(P3U1) and NS-I.

Subsequently, the aforementioned myeloma cells and antibody-producing cells are fused In cell fusion, antibody-producing cells (1 x 10⁶ to 1 x 10⁷ cells/ml) are mixed with myeloma cells (2 x 10⁵ to 2 x 10⁶ cells/ml) in an animal cell culture medium containing no serum, such as DMEM or RPMI-1640 medium (the cell ratio between the antibody-producing cells and the myeloma cells is preferably 5 : 1), and a fusion reaction is carried out in the presence of a cell fusion promoter. As such a cell fusion promoter, polyethylene glycol having a mean molecular weight of 1000 to 6000 daltons or the like can be used. Moreover, antibody-producing cells can also be fused with myeloma cells using a commercially available cell fusion apparatus that utilizes electrical stimulation (for example, electroporation).

Hybridomas of interest are selected from the cells that have undergone cell fusion. As a selection method, a cell suspension is diluted, as appropriate, with a fetal bovine serum-containing RPMI-1640 medium or the like, and it is then dispersed on a microtiter plate in a concentration of approximately 3 x 10⁵ cells/well. Thereafter, a selective medium is added to each well, and a culture is then carried out while appropriately exchanging the medium with a fresh one. As a result, cells that grow approximately 14 days after initiation of the culture can be obtained as hybridomas.

Subsequently, screening is carried out to examine the presence or absence of an antibody of interest in a culture supernatant of such growing hybridomas. The screening of hybridomas may be carried out by an ordinary method. Thus, the type of the screening method is not particularly limited. For example, an aliquot of a culture supernatant contained in a well in which hybridomas have grown may be collected, and it may be then screened by an enzyme immunoassay (ELISA, etc.), a radioimmunoassay, or the like. In the present invention, screening is carried out by a method of sensitively reflecting the coupling constant of an antibody, so as to reliably select hybridomas that produce useful antibodies. Such a method of reflecting the coupling constant of an antibody can be carried out by allowing an antibody and an antigen during the reaction to come into contact with an antigen protein or an antigen derivative in a concentration at the same level as, or higher or lower by an order of approximately 1 or 2 than the reciprocal of a target coupling constant (for example, when such a target coupling constant is $10^9$ M$^{-1}$, each antigen is allowed to come into contact with each antigen protein or an antigen derivative of approximately $10^{-10}$ M to $10^{-6}$ M, and more preferably of approximately 1 $0^{-9}$M~$10^{-7}$M), and by combining this method with a measurement method having sensitivity sufficient therefor.

The cloning of the fused cells is carried out by a limiting dilution method or the like, and hybridomas can be finally established as monoclonal antibody-producing cells.

As a method of collecting monoclonal antibodies from the established hybridomas, a common cell culture method, an ascites formation method, and the like can be adopted. In the cell culture method, hybridomas are cultured in an animal cell culture medium such as a 10% fetal bovine serum-containing RPNU- 1640 medium, an MEM medium, or a serum free medium, under general culture conditions (for example, 37°C, 5% $CO_2$ concentration) for 7 to 14 days. Thereafter, antibodies are obtained from a culture supernatant.

In the case of the ascites formation method, approximately 1 x $10^7$ hybridomas are administered into the abdominal cavity of an animal of the same species as an mammal from which myeloma cells are derived, so that large quantities of hybridomas are allowed to proliferate therein. One or two weeks later, ascites is collected. When it is necessary to purify antibodies in the aforementioned method of collecting antibodies, the collected antibodies can be purified by appropriately selecting a method from known purification methods such as ammonium sulfate fractionation, ion exchange chromatography, gel filtration, and affinity chromatography (protein A-aragose, etc.), or by applying these purification methods in combination.

Examples of an antibody subclass include IgG1, GgG2a, and IgG2b. The subclass used in the present invention is IgGI exhibiting good antibody fragmentation efficiency when an enzyme-antibody complex is produced. The obtained IgGI may be converted to F(ab')$_2$ by eliminating its Fc portion with activated papain, pepsin, etc. The F(ab')$_2$ may be further reduced, so that it can be converted to a Fab' fragment.

In a preferred embodiment, a Fab' fragment is used as a monoclonal antibody. An intact antibody (IgG) has Fab (an antigen-binding site) and Fc (a complement-binding site). When an intact antibody is allowed to bind to an enzyme, and the obtained product is used as an enzyme-labeled antibody, if a sample is a blood sample, a complement component in the blood may bind to an Fc portion, thereby causing steric hindrance and inhibiting enzyme activity. Even in a case in which a sample is not a blood sample, since such Fc portions are non-specifically adsorbed on the pores of a porous member or the surfaces of internal voids that constitute the wall or immune reaction layer of a reactor, the activity of an enzyme-labeled antibody becomes apparently low, and this causes noise during the measurement. To eliminate such noise, it is desired to use an Fab', F(ab')$_2$, or Fab fragment containing no Fc portions as an antibody. Of these, an Fab' fragment having a free SH group is most preferably used as an antibody, in terms of convenience for binding to enzyme.

The aforementioned monoclonal antibody of the present invention, or a fragment of the aforementioned monoclonal antibody, which is labeled with an enzyme (for example, *Bacillus subtilis* $\alpha$-amylase, etc.), can be used as an immunoanalytical reagent or kit for the measurement of canine CRP.

Such an enzyme used as a labeling substance can be selected, while taking into account a combination of the enzyme with an enzyme substrate used in the subsequent enzyme reaction. In the present invention, reactivity with an enzyme reacting with an enzyme substrate is suppressed by steric hindrance caused by formation of a matrix-like structure consisting of an enzyme, an antibody, and an antigen. Thus, as a combination of an enzyme with a substrate, a system for easily detecting the influence of such steric hindrance is preferably selected. That is to say, in terms of sensitivity, a relatively high-molecular-weight enzyme substrate is preferable. For example, a substrate with a molecular weight of approximately 20,000 or more, and more preferably of approximately 100,000 or more, is used. Such a substrate that binds to amylase is starch; such a substrate that binds to cellulase is cellulose; such substrates that bind to protease are proteins such as gelatin or hemocyanin; and such substrates that bind to lipase are various types of lipids. JP Patent Publication (Kokai) Nos. 60-108756 A (1985), 60-171461 A (1985), and 60-171460 A(1985) disclose in detail selection of the aforementioned enzyme and substrate. Among the aforementioned combinations of enzymes with substrates, a combination of amylase and starch as an enzyme substrate is preferable. In addition, water-insoluble substrates are particularly preferably used because the steric hindrance caused by a matrix-like structure consisting of an enzyme, an antibody, and an antigen is prominently manifested.

Examples of amylase include $\alpha$-amylase, $\beta$-amylase, and glucoamylase. Amylase that is not substantially contained in a sample is preferable in terms of noise prevention. The origin of such amylase varies widely from animals (saliva, pancreatic juice, etc.) and plants to microorganisms. Thus, when the body fluid, blood, or the like of a human or animal is analyzed, it is preferable not to use amylase derived from such a higher animal.

Examples of amylases derived from microorganisms or plants include: glucoamylases derived from Aspergillus sp., Rhizopus sp., Saccharomyces sp., etc.; $\beta$-amylase derived from barley malt, wheat, soybean, etc.; and $\alpha$-amylases derived from *Bacillus Subtilis, Streptomyces griseus, Pseudomonas stutzeri, Thermoactiomyces vulgaris,* etc. Of these, $\alpha$-amylase derived from *Bacillus Subtilis* is most preferable because it has high liquefying power and excellent heat stability.

These enzymes are preferably not affected by interfering factors existing in all analytes. In addition, it is preferable that no competitive enzymes of the same species be present in an analyte. If an enzyme of the same species as a labeling enzyme were contained in an analyte, an enzyme inhibitor might be used. The level of such an enzyme inhibitor to inhibit an enzyme contained in an analyte may be greater than the level thereof to inhibit the activity of a labeling enzyme. An enzyme inhibitor that completely deactivates an enzyme contained in an analyte but that does not inhibit at all a labeling enzyme is most preferable. From a practical viewpoint, however, it is sufficient if a blank value is not increased by such an enzyme inhibitor during the measurement. Thus, it is acceptable even if an enzyme inhibitor is deactivated and the activity of enzyme contained in a sample is recovered after the measurement. The type of such an enzyme inhibitor is not particularly limited, as long as it does not inhibit the enzyme of an enzyme-labeled antibody. It is acceptable even if such an enzyme inhibitor inhibits an enzyme in a free state. As such an enzyme inhibitor, that having the aforementioned specificity may be selected from known enzyme inhibitors and may be used. Otherwise, an antibody reacting with problematic enzyme contained in an analyte may be produced, and it may be used as an enzyme inhibitor.

When amylase is used as a labeling enzyme, $\alpha$-amylase is present as a competitive enzyme of same species in an analyte. Accordingly, it is preferable to inhibit the amylase present in the analyte using an enzyme inhibitor. The type of such an inhibitor that inhibits the amylase present in the analyte is not particularly limited, as long as the level of inhibiting the enzyme present in the analyte is greater than the level of inhibiting the activity of a labeling enzyme.

In particular, in the case of canines, there are a considerable number of analytes having an $\alpha$-amylase activity of 2500 U/L or more. Thus, an inhibitor that effectively inhibits an enzyme at a low dose is preferable.

It is said that the specificity of such an amylase inhibitor is different depending on the origin thereof. There are amylase inhibitors from bush bean, wheat, bacteria and the like. Of these, a bacteria-derived amylase inhibitor is preferable. For example, an amylase inhibitor from *Streptomyces nitrosporeus* specifically inhibits $\alpha$-amylase mainly from higher animals.

Examples of such an amylase inhibitor include, but are not limited to, $\alpha$-Amylase Inhibitor (manufactured by CALZYME Laboratories, Inc.), $\alpha$-Amylase Inhibitor from *Triticunn aestivum* (wheat seed) (manufactured by Sigma), and $\alpha$-Amylase Inhibitor (from *Streptomyces nitrosporeus)* (manufactured by Wako Pure Chemical Industries, Ltd.).

When $\alpha$-amylase is used as an enzyme, carboxymethylated starch, starch, amylose, amylopectin, or the like may be used as a substrate. In particular, if water-insoluble starch or the like is used, an enzyme reaction takes place on the surface of a substrate particle, namely, on the interface between solid and liquid. As a result, the influence of steric hindrance due to an antibody-antigen bond upon enzyme activity appears significantly. Thus, the use of such water-insoluble starch is preferable in terms of sensitivity. Moreover, water-insoluble dye starch may be used to detect dye (pigment) adhering to soluble amylose as an enzyme decomposed product. As such water-insoluble blue starch polymer, a commercially available product such as Neo-Amylase (manufactured by Daiichi Kagaku Yakuhin Co.) can be used.

A method of binding an enzyme with an antibody can be carried out utilizing the functional groups of the two substances (an amino group, a carboxyl group, a thiol group, etc.). Representative binding methods include a glutaraldehyde method, a periodic acid method, a pyridyl disulfide method, and a maleimide-succinimide method. However, examples of such a binding method are not limited thereto, In addition to the aforementioned methods, a method may be selected, as appropriate, from those described in publications such as "Method in Immunology and Immunochemistry," Vol. 1, C. A. Williams, M. W. Chase, Academic Press, 1967, or "Koso Meneki Sokutei Ho (Enzyme Immunoassay)" edited by Ishikawa, Kawai, and Miyai, Igaku-Shoin, Ltd., 1978, and it may be then used. Among these binding methods, a maleimide-succinimide method comprising binding a thiol group of an antibody hinge portion to an amino group of enzyme via crosslinkage is superior in terms of good reaction efficiency and ability to maintain antibody activity.

In the maleimide-succinimide method, an enzyme is allowed to bind to Fab', for example, as follows. First, an amino group of the enzyme is maleimidated with a maleimide-succinimide reagent. The resultant is purified by gel filtration, and it is then combined with an antibody having a thiol group (Fab'). In this complex formation, two or more types of antibodies having different epitopes (Fab') may be used in combination. In such a case, such antibody fragments are also subjected to a binding reaction. This complex formation reaction is preferably carried out using 3 to 7 moles of antibodies with respect to 1 mole of enzyme. When Fab' (molecular weight: approximately 50,000) is used as an antibody and $\alpha$-amylase (molecular weight: 50,000) is used as an enzyme, a binding reaction is preferably carried out using 1/3 to 1/7 by weight of $\alpha$-amylase with respect to the total weight of Fab'. This binding reaction is generally carried out at 4°C to room temperature.

The generated enzyme-antibody complex (enzyme-labeled antibody) is purified by gel filtration, and as necessary, it is then freeze-dried. The binding ratio between an enzyme and each antibody is not limited to 1 : 1, and any given ratio can be applied depending on purposes. Since an enzyme generally has a large number of amino groups, multiple maleimide groups are introduced, and multiple antibody molecules are introduced into a single enzyme molecule. Since at least one antibody molecule should bind to one enzyme molecule, the molar ratio of such an antibody to such an enzyme in a complex should be 1 or greater. In order to reliably enhance detection sensitivity, such a molar ratio is preferably set at 2 to 5, and is more preferably set at 2 to 3. When Fab' (molecular weight: approximately 50,000) is used as an antibody and $\alpha$-amylase (molecular weight: 50,000) is used as am enzyme, from the viewpoint of high detection sensitivity, the molecular weight of a complex is 100,000 daltons or more, preferably 200,000 to 500,000 daltons, and most preferably 200,000 to 300,000 daltons.

The analytical element for the measurement of canine CRP of the present invention uses the above-described monoclonal antibody prepared by labeling, with an enzyme, a first monoclonal antibody fragment that recognizes canine CRP, and it further uses a second monoclonal antibody. The second monoclonal antibody that recognizes canine CRP has a coupling constant of $10^7$ M$^{-1}$ to $10^9$ M$^{-1}$ with respect to canine CRP, and it is of the IgG1 subclass. The second monoclonal antibody may be either identical to or different from the first monoclonal antibody, but it is neither a fragment of an antibody nor a labeled antibody.

The used amounts of the labeled first monoclonal antibody and the second monoclonal antibody, which are used in the analytical element for the measurement of canine CRP of the present invention, may be appropriately changed. However, the molar ratio between the two antibodies is preferably 1 to 20. The molar ratio is most preferably 5 to 15. If the amount of the second monoclonal antibody is excessively larger than that of the labeled first monoclonal antibody, the second monoclonal antibody binds to a majority of canine CRP, and thereby the labeled first monoclonal antibody does not effectively act, so that detection sensitivity cannot be obtained. In contrast, if the amount of the second monoclonal antibody is too small to that of the labeled first monoclonal antibody, the labeled first monoclonal antibody reacts with canine CRP to a certain extent, but a dynamic range cannot be obtained.

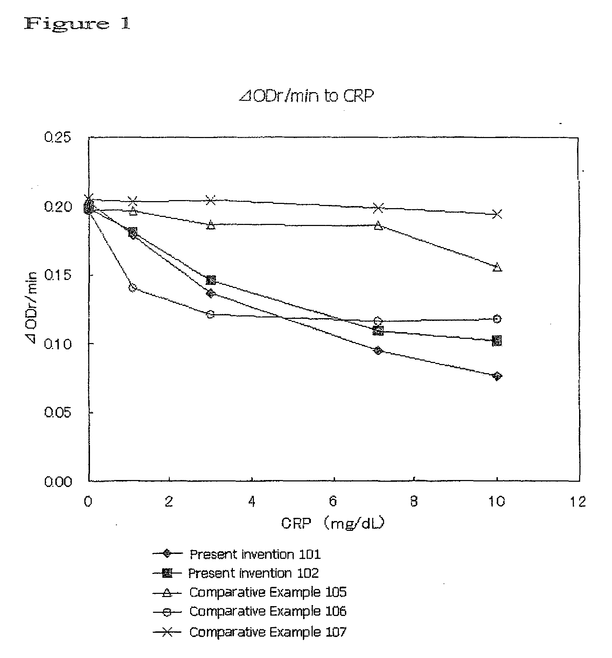

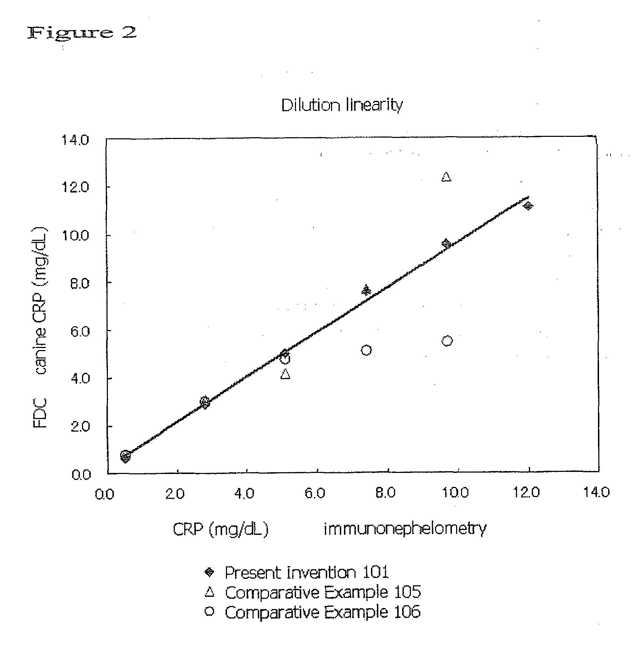

Next, the dry analytical element for the measurement of canine CRP of the present invention will be described. Specific examples of the configuration of such a dry analytical element include those shown in Figures 1 and 2 of Japanese Patent No. 3151080.

That is to say, as an example, a detection layer (or a reagent layer) and an immune reaction layer are laminated on a light-transmittable support. The immune reaction layer is composed of a water-penetrable layer, and it contains the enzyme-labeled antibody of the present invention and a non-diffusible substrate used as a substrate of a labeling enzyme. The reagent layer is composed of a water-penetrable layer, and it contains a reagent composition for detecting an enzyme reaction product (a diffusible substance) dispersed and moved from the immune reaction layer. When such an enzyme reaction product is a substance that can be directly detected, such as a colored substance, it is not necessary for the detection layer (or reagent layer) to contain a reagent composition used in detection. In this case, the detection layer (or reagent layer) functions as a detection layer.

An analyte (an antigen) contained in a liquid sample supplied to the element reacts with an enzyme-labeled antibody via an antigen-antibody bond in the immune reaction, layer so as to form a matrix structure. Thus, enzyme activity on a substrate contained in the same immune reaction layer is suppressed. As a result, the amount of the antigen contained in the analyte can be measured based on the amount of an enzyme reaction product detected in the reagent layer (or detection layer).

Moreover, as another example, an enzyme-labeled antibody and an enzyme substrate may be added to different layers. In this case, a water-penetrable substrate layer containing an enzyme substrate is disposed on a reagent layer (or a detection layer), and an immune reaction layer containing an enzyme-labeled antibody is further disposed thereon. In this case, an analyte (an antigen) contained in a liquid sample supplied to the element reacts with the enzyme-labeled antibody via an antigen-antibody bond in the immune reaction layer to form a matrix structure, so that the analyte becomes substantially immovable. An enzyme-labeled antibody that has not bound to the antigen (or a matrix structure that is small enough not to be captured by a layer structure) moves to the subsequent substrate layer.

In any one of the aforementioned embodiments, an enzyme immune reaction can be promoted in the element only by adding a liquid sample to the element

Furthermore, the dry analytical element of the present invention may be prepared as a multilayer comprising an immune reaction layer (or an immune reaction layer and a substrate layer), a reagent layer (or a detection layer), a support, a spreading layer, a detection layer, a light shielding layer, an adhesion layer, a water absorption layer, an undercoating layer, and other layers. Such analytical elements are disclosed, for example, in the specifications of JP Patent Publication (Kokai) No. 49-53888 A (1974) (the corresponding US Patent No. 3,992,158), JP Patent Publication (Kokai) No. 51-40191 A (1976) (the corresponding US Patent No. 4,042,335), JP Patent Publication (Kokai) No. 55-164356 A (1980) (the corresponding US Patent No. 4,292,272), and JP Patent Publication (Kokai) No. 61-4959 A (1986) (the corresponding EPC Laid-Open Patent No. 0166365A).

When a light-transmittable water-impenetrable support is used, the dry immunoanalytical element of the present invention may practically have the following structures. However, the contents of the present invention are not limited to the following examples.

(1) A structure having a reagent layer on a support and further having an immune reaction layer thereon.
(2) A structure having a reagent layer, a substrate layer, and an immune reaction layer on a support in this order.
(3) A structure having a reagent layer, an adhesion layer, and an immune reaction layer on a support in this order.

(4) A structure having a reagent layer, an adhesion layer, a substrate layer, and an immune reaction layer on a support in this order.

(5) A structure having a detection layer, a reagent layer, and an immune reaction layer on a support in this order.

(6) A structure having a detection layer, a reagent layer, a substrate layer, and an immune reaction layer on a support in this order.

(7) A structure having a reagent layer, a light reflection layer, and an immune reaction layer on a supporting this order.

(8) A structure having a reagent layer, a light reflection layer, a substrate layer, and an immune reaction layer on a support in this order.

(9) A structure having a detection layer, a reagent layer, a light reflection layer, and an immune reaction layer on a support in this order.

(10) A structure having a detection layer, a reagent layer, a light rejection layer, a substrate layer, and an immune reaction layer on a support in this order.

(11) A structure having a detection layer, a light reflection layer, a reagent layer, and an immune reaction layer on a support in this order.

(12) A structure having a detection layer, a light reflection layer, a reagent layer, a substrate layer, and an immune reaction layer on a support in this order.

(13) A structure having a second reagent layer, a light reflection layer, a first reagent layer, and an immune reaction layer on a support in this order.

(14) A structure having a second reagent layer, a light reflection layer, a first reagent layer, a substrate layer, and an immune reaction layer on a support in this order.

(15) A structure having a detection layer, a second reagent layer, a light reflection layer, a first reagent layer, and an immune reaction layer on a support in this order.

(16) A structure having a detection layer, a second reagent layer, a light reflection layer, a first reagent layer, a substrate layer, and an immune reaction layer on a support in this order.

In the structures described in (1) to (12) above, the reagent layer may consist of multiple different layers. A water absorption layer may be established between a support and a reagent layer or a detection layer. In addition, a filtration layer may be established between individual layers. Moreover, a spreading layer may be established on a substrate layer, or spreading action may be imparted to the substrate layer so that the layer can function as a spreading layer. When a solid component such as a hemocyte is contained in an analyte, a suitable filtration layer may be established as the uppermost layer of the analytical element.

Either an immune reaction layer or a substrate layer is composed of a water-penetrable layer. To secure the water penetrability of such a layer, such a layer is preferably prepared as a porous layer consisting of a porous medium or as a layer consisting of a hydrophilic polymer binder.

Such a porous layer may be either fibrous or non-fibrous. As a fibrous material, a filter, a non-woven fabric, a woven fabric (e.g. a plain-woven fabric), a knitted fabric (e.g. a tricot knitted fabric), a glass fiber filter, etc. can be used, for example. As a non-fibrous material, a membrane filter consisting of cellulose acetate or the like described in JP Patent Publication (Kokai) No. 49-53888 (1974) A, a continuous void-containing granular structure layer consisting of inorganic or organic fine particles described in JP Patent Publication (Kokai) No. 49-53888 (1974) A, JP Patent Publication (Kokai) No. 55-90859 (1980) A (the corresponding US Patent No. 4,258,001), and JP Patent Publication (Kokai) No. 58-70163 (1983) A (the corresponding US Patent No. 4,486,537), and the like may be used. A lamination layer product consisting of multiple porous layers that are partially adhered to one another described in JP Patent Publication (Kokai) No. 61-4959 (1986) A (the corresponding European Paten Application Laid-Open No. EP 0166365A), JP Patent Publication (Kokai) No. 62-116258 (1987) A, JP Patent Publication (Kokai) No. 62-138756 (1987) A (the corresponding European Paten Application Laid-Open No. EP 0226465A), JP Patent Publication (Kokai) No. 62-138757 (1987) A (the corresponding European Paten Application Laid-Open No. EP 0226465A), JP Patent Publication (Kokai) No. 62-138758 (1987) A (the corresponding European Paten Application Laid-Open No. EP 0226465A), etc. is also preferably used.

Such a porous layer may be a spreading layer having what is called "measuring action" to spread liquid on an area that is almost proportional to the amount of the liquid to be supplied. As such a spreading layer, a woven fabric, a knitted fabric, or the like is preferable from among the aforementioned examples. A glow discharge treatment as described in JP Patent Publication (Kokai) No. 57-66359 (1982) A may be performed on a woven fabric. In order to control a spreading area, a spreading rate, etc., a spreading layer may comprise a hydrophilic polymer or a surfactant as described in JP Patent Publication (Kokai) No. 60-222770 (1985) A (the corresponding EP 0162301A), JP Patent Publication (Kokai) No. 63-219397 (1988) A (the corresponding West German Patent Application Laid-Open No. DE 37 17 913A), JP Patent Publication (Kokai) No. 63-112999 (1988) A (the corresponding DE 37 17 913A), and JP Patent Publication (Kokai) No. 62-182652 (1987) A (the corresponding DE 3717 913A).

An example of a useful method of adding a substrate to a porous layer is a method, which comprises: previously impregnating or coating a porous membrane or the like consisting of a paper, a fabric or a polymer with a substrate; and adhering the porous membrane onto another water-penetrable layer such as a reagent layer established on a support according to the method described in JP Patent Publication (Kokai) No. 55-164356 (1980) A. There may be an alternative method, which comprises adhering a porous layer to another water-penetrable layer (e.g. a reagent layer) according to the aforementioned method, and then coating the porous layer with a composition containing a substrate. Such a porous layer may be impregnated or coated with a composition containing a substrate by known methods. As such a coating method, dipping coating, doctor coating, hopper coating, curtain coating, or the like may be selected, as appropriate, and may be then used. The thickness of the thus produced substrates layer is not particularly limited. When such a substrate layer is established as a coating layer, the thickness of the layer is approximately 1 to $50\mu$ m, and preferably 2 to 30 $\mu$m. When such a substrate layer is produced by methods other than a coating method, such as lamination with a laminate, the thickness of the layer may vary largely from several tens of $\mu$m to several hundreds of $\mu$m.

When an immune reaction layer and a substrate layer are constituted with a water-penetrable layer consisting of a hydrophilic polymer binder, the following materials may be used as hydrophilic polymers: gelatin and a derivative thereof (e.g. phthalic gelatin), a cellulose derivative (e.g. hydroxyethylcellulose), agarose, sodium alginate, an acrylamide copolymer, a methacrylamide copolymer, a copolymer of acrylamide or methacrylamide with various types of vinyl monomers, polyhydroxyethyl methacrylate, polyvinyl alcohol, polyvinylpyrrolidone, sodium polyacrylate, and a copolymer of acrylic acid with various types of vinyl monomers.

A substrate layer constituted with a hydrophilic polymer binder can be formed by coating a support or another layer such as a detection layer with an aqueous solution or a water dispersion comprising a substrate, a reagent composition, and a hydrophilic polymer according to the methods described in the specifications of JP Patent Publication (Kokoku) No. 53-21677 (1978) B (the corresponding US Patent No. 3,992,158), JP Patent Publication (Kokai) No. 55-164356 (1980) A (the corresponding US Patent No. 4,292,272), JP Patent Publication (Kokai) No. 54-101398 (1979) A (the corresponding US Patent No. 4,132,528), JP Patent Publication (Kokai) No. 61-292063 (1986) A (Chemical Abstracts, 106 210567y), etc., and then drying it. The thickness of a substrate layer comprising a hydrophilic polymer as a binder in a dry state is between approximately 2 and 50 $\mu$m, and preferably between approximately 4 and 30 $\mu$m. The coating amount is between approximately 2 and 50 g/m$^2$, and preferably between approximately 4 and 30 g/m$^2$.

For the purpose of improving coating property and various properties of a diffusible compound, such as dispersibility, reactivity and preserving property, a substrate layer may also comprise various types of organic or inorganic additives such as an enzyme activator, a coenzyme, a surfactant, a pH buffer composition, fine particles, and an antioxidant, in addition to a non-diff-usible substrate. Examples of a buffer that may be contained in the substrate layer include pH buffer systems described in "Kagaku Binran Kiso Hen (Chemistry Handbook, Basic Version)" edited by the Chemical Society of Japan, Tokyo, Mazuren Co., Ltd., 1966, pp. 1312-1320; "Data for Biochemical Research" edited by R. M. C. Dawson et al., 2nd edition, Oxford at the Clarendon Press, 1969, pp. 476-508; Biochemistry, 5, pp. 467-477, 1966; and Analytical Biochemistry, 104, pp. 300-310, 1980. Specific examples of such a pH buffer include: a buffer comprising Tris(hydroxymethyl)aminomethane (Tris); a buffer comprising phosphate; a buffer comprising borate; a buffer comprising citric acid or citrate; a buffer comprising glycine; a buffer comprising Bicine; a buffer comprising HEPES; and a Good's buffer such as a buffer comprising MES.

An immune reaction layer may have the same structure as that of the aforementioned substrate layer. In order to add a substrate and an enzyme-labeled antibody to a single layer or two layers adjacent to each other in a substantially dry state or in the substantially absence of water, such an enzyme-labeled antibody may be dissolved or dispersed in a non-aqueous solvent such as alcohol (e.g. ethanol), and thereafter, a water-penetrable layer may be impregnated with the non-aqueous solvent

A reagent layer comprises a reagent composition for detecting a diffusible substance dispersed and moved from the immune reaction layer (or the substrate layer). As necessary, such a reagent composition comprises a depolymerizing enzyme, and comprises a detection reagent composition for detecting a low-molecular-weight product generated as a result of reduction in the molecular weight of a diffusible substance. The reagent layer is constituted with a water-penetrable layer. It is preferably a continuous layer consisting of a hydrophilic polymer binder, from among the water-penetrable layers described in the above substrate layer. The type of a hydrophilic polymer binder used is determined, while taking into consideration a diffusible product generated in the substrate layer or a coloring reagent contained in the reagent layer.

When a diffusible substance dispersed and moved from the immune reaction layer (or the substrate layer) can be directly detected, it is unnecessary to add a detection reagent composition to the reagent layer. The reagent layer functions as a detection layer. Even if the reagent layer functions as a detection layer, it is preferably a continuous layer consisting of a hydrophilic polymer binder, from among the water-penetrable layers described in the above substrate layer.

Any one of light non-transmittable (opaque), light semi-transmittable (semi-transparent), and light transmittable (transparent) supports can be used as a support. In general, a light transmittable water non-penetrable support is preferable. Preferred materials of such a light transmittable water non-penetrable support include polyethylene terephthalate and polystyrene. In order to strongly adhere a hydrophilic layer to the support, in general, an undercoating layer is established, or a hydrophilic treatment is performed on the support

As with the inventions described in JP Patent Publication (Kokai) No. 3-295466 (1991) A (the corresponding EP 0451848A), JP Patent Publication (Kokai) No. 4-128655 (1992) A, and JP Patent Publication (Kokai) No. 4-2765551 (1992) A, in the dry immunoanalytical element of the present invention, an increase in sensitivity can be achieved by adding, to a reagent layer, a depolymerizing enzyme that further depolynzerizes a diffusible substance decomposed from a non-diffusible substrate by a labeled enzyme. A combination of a labeled enzyme, a non-difusible substrate, a diffusible substance, and a depolymenzing enzyme, may be selected from combinations of these components, in which an enzyme acts on a non-diffusible substrate to generate a diffusible substance, and a depolymerizing enzyme as described later acts on the diffusible substance so as to convert it to a lower molecular weight product, which can be easily detected,

That is, as an antibody-labeling enzyme, there is selected an enzyme that decomposes a high-molecular-weight non-diffusible substrate to generate a diffusible product that is further converted to a lower molecular weight product by action of a depolymerizing enzyme. As a non-diffusible substrate, there is selected a substrate that is non-diffusible (insoluble) in an aqueous analyte solution and that is neither dispersed nor moved from an immune reaction layer (or a substrate layer) to a reagent layer. As a depolymerizing enzyme, there is selected an enzyme that depolymerizes a diffusible product generated from a non-diffusible substrate by the action of an antibody-labeling enzyme so as to convert it to a detectable low-molecular-weight product. Specific examples of such a depolymerizing enzyme will be given below.

As such an enzyme, a catabolic enzyme that produces a diffusible oligomer from a non-diffusible substrate consisting of a polymer is preferable. Among the aforementioned labeling enzymes, a saccharide-hydrolyzing enzyme is preferable, for example. Examples of such a saccharide-hydrolyzing enzyme include α-amylase, β-amylase, glucoamylase, and lysozyme.

Examples of a substrate reacting with the aforementioned α-amylase, β-amylase, and glucoamylase include carboxymethylated starch and starch. When such carboxymethylated starch or starch is used as a non-diffusible substrate, the following combination may be applied. That is, α-amylase is used as a labeling enzyme and glucoamylase or α-glucosidase as described below is used as a depolymerizing enzyme.

Such a depolymerizing enzyme may be of the same type as that of a labeling enzyme. In this case, the labeling enzyme is an endo-active enzyme that cleaves the inside of a molecule to produce an oligomer. The polymerizing enzyme is preferably an enzyme having exo activity to act on the end of a molecule to produce a monomer. In a case in which a non-diffusible substrate is a polymer (e.g. starch) for example, an enzyme that is able to decompose a diffusible oligomer (e.g. maltose) produced by a labeling enzyme into a monomer (e.g. glucose) is used as a depolymerizing enzyme. An examples of such a depolymerizing enzyme is saccharide-hydrolyzing enzyme. More specifically, α-amylase, β-amylase, glucoamylase, and α-glucosidase may be used. When carboxyl methyl cellulose and cellulase are used as a non-diffusible substrate and a labeling enzyme, respectively, C1 enzyme can be used as a depolymerizing enzyme.

As a combination of such a labeling enzyme, non-diffusible substrate, and depolymerizing enzyme, they can be selected from enzymes and substrates described in known publications (for example, "Koso Handbook (Enzynrze Handbook)," Bunji Maruo, Nobuo Tamiya, Asakura Publishing Co., Ltd., 1982, and "Seikagaku Handbook (Biochemical Handbook)" Nobumasa Imura, et al., Maruzen Co., Ltd.,1984).

A low-molecular-weight product produced by depolymerizing enzyme in a reagent layer can be optically detected using a known detection system reagent. As a method of detecting glucose finally generated by the aforementioned depolymerizing enzyme, the following known methods may be applied: a method comprising oxidizing glucose in the presence of glucose oxidase and detecting the generated hydrogen peroxide (for example, a method using the following reagent: a Trinder reagent described in Ann. Clin. Biochem., 6, 24 (1964); a Trinder reagent described in J. Clin. Pathol., 22, 246 (1969); Trinder reagents described in JP Patent Publication (Kokai) No. 49-50991 (1974) A (the corresponding US Patent No. 3,886,045), US Patent No. 3,992,158, JP Patent Publication (Kokai) No. 55-164356(1980) A (the corresponding US Patent No. 4,292,272), etc.; reagents comprising triaryl-substituted imidazole leuco dye described in JP Patent Publication (Kokai) No. 53-26188 (1978) A (the corresponding US Patent No. 4,089,747), JP Patent Publication (Kokai) No. 58-45557 (1983) A, etc.; or reagents comprising diaryl-monoaralkyl-substituted imidazole leuco dye described in JP Patent Publication (Kokai) No. 59-193352 (1984) A (the corresponding EP 0122641A), JP Patent Publication (Kokai) No. 60-224677 (1985) A (the corresponding US Patent No. 4,665,023), etc.); a method of detecting NADH generated in the presence of in the presence of glucose dehydrogenase and NAD; and a method of detecting glucose-6-phosphate generated in the presence of hexokinase. Among these detection methods, a method comprising oxidizing glucose in the presence of glucose oxidase and detecting the generated hydrogen peroxide using peroxidase and leuco dye is most preferable in terms of sensitivity.

These detection reagents may be contained in the reagent layer of the analytical element together with a depolymedzing enzyme. However, it may also be possible to add such a detection reagent to another layer established under the reagent layer (for example, the second reagent layer, the detection layer, etc.) so as to carry out detection in this layer. When leuco dye is used, such leuco dye is preferably dispersed in a hydrophilic polymer in a solution of a water-nonmiscible solvent from the viewpoint of the stability of the generated dye.

The dry immunoanalytical element of the present invention can be prepared by known methods described in the aforementioned patent specifications. From the viewpoint of production, wrapping, transportation, preservation, measurement operations, etc., it is preferable that the analytical element of the present invention be cut into a small piece such as a square with a side of approximately 15 mm to approximately 30 mm or a circle having almost the same above size, and that it be then placed into the slide frame described in JP Patent Publication (Kokoku) No. 57-28331 (1982) B (the corresponding US Patent No. 4,169,751), JP Utility Model Publication (Kokai) No. 56-142454 (1981) U (the corresponding US Patent No. 4,387,990), JP Patent Publication (Kokai) No. 57-63452 (1982) A, JP Utility Model Publication (Kokai) No. 58-32350 (1983) U, JP Patent Publication (Kohyo) No. 58-501144 (1983) A (the corresponding International Publication WO83/00391 or the like, so that it is used as a chemical analysis slide. Depending on intended use, the analytical element of the present invention may be processed into a long tape, which may be placed in a cassette or a magazine and may be then used. Otherwise, a small piece thereof may be attached to or placed in a card with an aperture and may be then used.

By carrying out the same operations as those described in the aforementioned patent specifications, the dry analytical element of the present invention can be used in the quantitative analysis of a high-molecular antigen that is a test substance contained in a liquid sample. For example, approximately 5 to 30 $\mu$L of, and preferably 8 to 15 $\mu$L of an aqueous liquid sample solution such as plasma, serum, or urine is added to a substrate layer. The resultant analytical element is incubated at a constant temperature between approximately 20°C and approximately 45°C, and preferably between approximately 30°C and approximately 40°C, for 1 to 10 minutes. Color development or discoloration occurring in the element is measured from the side of a light transmittable support via reflection photometry. Thereafter, the amount of the high-molecular antigen in the analyte can be obtained by the principle of colorimetry using the previously prepared calibration curve. The amount of a liquid sample added, the incubation time, and the temperature are kept constant, so that quantitative analysis can be carried out with high accuracy. Using chemical analysis apparatuses described in JP Patent Publication (Kokai) Nos. 60-125543 (1985) A, 60-220862 (1985) A, 61-294367 (1986) A, etc., highly accurate quantitative analysis can be carried out by extremely easy operations. Depending on purposes or required accuracy, the degree of color development may be determine by visual observation, and semi-quantitative measurement may be earned out When the analytical element comprises no enzyme-labeled antibodies, an aqueous sample solution may be mixed with a solution containing an enzyme-labeled antibody before adding it to the element, so that a binding reaction may be sufficiently carried out. Thereafter, the reaction solution may be added to the substrate layer.

The present invention further relates to a method for measuring CRP in a sample, which comprises allowing a sample to come into contact with the aforementioned immunoanalytical reagent for measuring canine CRP and human CRP of the present invention, or the dry analytical element for measuring canine CRP and human CRP of the present invention. The type of a sample is not particularly limited. Examples of such a sample include blood (whole blood, plasma, and serum), lymph, and urine. A preferred example is blood (whole blood, plasma, and serum), more preferred examples are serum and plasma, and a particularly preferred example is serum.

The present invention will be specifically described in the following examples. However, these examples are not intended to limit the scope of the present invention.

Examples

Example 1: Preparation of hybridoma, which produces monoclonal antibody of IgGI subclass having reactivity with canine CRP

(1) Preparation of antibody-producing fused cells (hybridomas):

Canine CRP separated and purified from the fresh serum of a beagle canine using an affinity column was used as an antigen. Mice (BALBc) were immunized with the antigen. After confirming that an antibody titer with respect to canine CRP had increased, splenic cells were collected, and the collected splenic cells were then fused with myelomas (P3U1) by a PEG method.

The fused cells were subjected to selective culture in an HAT medium, so as to obtain a large number of proliferating hybridomas.

(2) Selection of hybridomas (1): Confirmation of antigenic specificity and confirmation of type and subclass of antibody

The protein antigen of canine CRP was diluted with PBS(-) so that the concentration is 1 $\mu$g/mL, and was immobilized on solid phase. The culture supernatant of the hybridomas obtained in (1) above was analyzed by an ELISA method on a microplate by using an anti-mouse IgG HRP-labeled goat antibody (Goat anti-mouse IgG (H+L) HRP conjugated secondary antibody; No. AP308P; Funakoshi Corp.) as a secondary antibody, so that the reactivity of the antibody with the antigen was confirmed. As a result, several types of hybridomas producing antibodies reacting with the canine CRP antigens could be selected. In addition, the isotypes and subclasses of antibodies in the culture supernatants of these hybridomas were identified using commercially available identification kit reagents.

(3) Selection of hybridomas (2): Confirmation of coupling constant to each antigen

With regard to a culture supernatant, which produced an antibody of IgG type, selected from among culture supernatant which had been confirmed to have reactivity with canine CRP, the coupling constant was measured using the apparatus of Biacore (Biacore3000). Rabbit anti-mouse IgG was immobilized on a sensor chip, and the sensor chip was then allowed to come into contact with the culture supernatant under certain conditions, so as to immobilize thereon mouse IgG existing in the culture supernatant. Subsequently, each antigen protein (4 $\mu$g/mL; 3.5 x $10^{-8}$M) was allowed to come into contact with the culture supernatant (a target coupling constant: $10^9$ M$^{-1}$). Using a signal increase occurring when the culture supernatant was allowed to come into contact (antibody signal: corresponding to the amount of IgG in the culture supernatants) as a baseline, a signal increase occurring when an antigen protein was allowed to come into contact (antigen signal: corresponding to the ability of an antibody to capture an antigen) was compared with the aforementioned signal increase as a baseline. Thus, the order of the coupling constants of antibodies contained in the culture supernatant can be assumed. The measurement results of the culture supernatant are shown in Table 1. Antibodies were prepared by a mouse ascites method using three types of hybridomas, 3A6, 11C6 and 16H4, and the prepared antibodies were then purified with a protein A column. The signal ratios of the thus obtained antibodies under the same conditions were well matched with those of culture supernatants. These results demonstrate that the results obtained using culture supernatants are effective for selection of antibodies. By this method, the hybridoma 16H4 could be selected as a hybridoma producing an antibody, which has a sufficient coupling constant ($10^7$M$^{-1}$ to $10^9$M$^{-1}$) with respect to canine CRP and whose subclass is IgG1. Moreover, the hybridomas 3A6 and 11 C6 could also be obtained.

The hybridoma 16H4 (identification number assigned by depositor: MM44097-16H4F5) was deposited with the National Institute of Advanced Industrial Science and Technology, an Independent Administrative Institution under the Ministry of Economy, Trade and Industry, at the AIST Tsukuba Central 6, Higashi 1-1-1, Tsukuba, Ibaraki, Japan, under accession No. FERM P-21571 (reception No. FERM AP-21571) on April 18, 2008. The above-mentioned hybridoma deposited under accession No. FERM P-21571 was then transferred to an international deposition under the provisions of the Budapest Treaty on May 27, 2009, and received an accession No. FER24 BP-11132.

Table 1: Results of microplate ELISA and Biacore measurement of various types of hybridoma culture supernatants and purified done culture supernatant

| | | ELISA color development OD*2 | Biacore measurement *3 Antigen signal/antibody signal | | |
| --- | --- | --- | --- | --- | --- |
| | | | Culture supernatant | Purified clone antibody | |
| Hybridoma name | Isotype/subclass | Canine CRP | Canine CRP | Canine CRP | Remarks |
| 5B8 | IgG2b | 2.752 | — | 0 | Compara Example |
| 8B5 | IgG2b | 2.955 | — | 0 | Compara Example |
| 8G11 * 1 | IgG1 | 1.487 | 0 | — | Compara Example |
| 3A6 | IgG1 | 0.111 | 0.368 | 0.555 | The present invention |
| 11C6 | IgG1 | 0.300 | 0.535 | 0.502 | The present invention |
| 16H4 | IgG1 | 0.706 | 0.887 | 0.882 | The present invention |
| *1: Eliminated during cloning process <br> *2: Antigen concentration during immobilization: 1 $\mu$g/mL <br> *3: Antigen concentration: 4 $\mu$g/mL (approximately 3.5 x $10^{-8}$ M) | | | | | |

Example 2: Preparation of monoclonal antibody

The hybridoma 16H4 was dissolved and cultured with RPMI 1640 + 10% FBS. Thereafter, 0.5 mL (5 x $10^6$ cells/mouse) of the thus proliferating cells was implanted into each of 20 mice. After a certain period of time, ascites was collected from the abdominal cavity and was then centrifuged. The supernatant was purified by the following protein A-affinity chromatography method. The supernatant of ascites diluted with 1.5 M glycine-NaOH (pH 8.9)-3 M NaCl was supplied to a protein A column equilibrated with the same above buffer, so that IgG was absorbed thereon. The adsorbed IgG was eluted with an elution buffer (100 mM citrate buffer; pH 3.0), and a neutralization solution (2 M Tris-HCl; pH 9.0) was immediately added thereto, so that the pH was adjusted to pH 7.0 to 7.5. The resultant was dialyzed against PBS, so as to obtain a purified antibody.

Example 3: Preparation of analytical element for the measurement of canine CRP

Table 2 shows the coupling constants of canine CRP antibodies with respect to canine CRP. As comparative example, the coupling constant of a human CRP antibody with respect to human CRP is also shown. As labeled antibodies, anti-canine CRP. IgGFab' antibodies were prepared by labeling the canine CRP antibodies shown in Table 1 with an enzyme, *Bacillus subtilis* α-amylase, according to a method equivalent to the method described in Japanese Patent No. 3151080. As a comparative example, anfi-human CRP- IgGFab' was also prepared by labeling a human CRP antibody with the enzyme. Using the materials shown in Table 2, analytical elements for the measurement of canine CRP were produced. As comparative example, there was produced an analytical element for the measurement of human CRP, in which a human CRP antibody was used. As the second antibodies, the canine CRP antibodies shown in Table 2 were used.

Table 2: Labeled canine CRP antibody and canine CRP antibody

| Sample No. | Antibody name | Coupling constant ($M^{-1}$) to canine CRP | Name of antibody labeled with enzyme | Remarks |
|---|---|---|---|---|
| 1 | A (16H4) | 2.5 x $10^8$ | LA | The present invention |
| 2 | B (3A6) | 1.5 x $10^8$ | LB | The present invention |
| 3 | C (11C6) | 1.4 x $10^8$ | LC | The present invention |
| 4 | D (5B8) | 1.0 x $10^4$ | LD | Comparative Example |

Production of dry analytical element for the measurement of CRP

A 180-μm colorless, transparent smooth film of polyethylene terephthalate which was undercoated with gelatin was coated with an aqueous solution of the following composition and dried to a thickness of 14 μm.

| | |
|---|---|
| Gelatin | 14.1 g/m$^2$ |
| Peroxidase | 12.0 KU/m$^2$ |
| Glucose oxidase | 6.0 KU/m$^2$ |
| Glucoamylase | 5.0 KU/m$^2$ |
| Leuco Dye | 0.5 g/m$^2$ |
| Surfactant | 1.0 g/m$^2$ |

As a surfactant, polyoxy(2-hydroxy)propylene nonylphenyl ether (Surfactant 10G; manufactured by Olin) was used, As a leuco dye, 2-(3,5-dimethoxy-4-hydroxyphenyl)-4-(4-dirnethylaminophenyl)-5-phenethylimidazole acetate was used.

Subsequently, the aforementioned film was coated with an aqueous solution of the following composition and dried to a thickness of 10 μm.

| | |
|---|---|
| Gelatin | 10.2g/m$^2$ |
| Surfactant | 0.5g/m$^2$ |

Subsequently, the aforementioned film was coated with an aqueous solution of the following composition, whose pH had been adjusted to pH 6.4, and it was then dried to a thickness of 8 μm.

| | |
|---|---|
| Hydroxypropylceilulose | 4.7 g/m² |
| Carboxymethyl starch | 3.5 g/m² |
| PIPES | 0.9 glm² |
| Mannitol | 2.3g/m² |
| Surfactant | 1.2g/m² |
| Amylase inhibitor | 1020KU/m² |

Thereafter, water was supplied in an amount of approximately 60 g/m² onto the entire surface of the aforementioned film so as to wet the film. A tricot knitted fabric knitted from 50 deniers polyethylene terephthalate spun yam by 36 gauges was laminated on the film by light compression, and it was then dried.

The aforementioned fabric was coated with ethanol to a concentration of 200 g/m² (= OC1 application), and it was then dried. Thereafter, the resultant fabric was further coated with an ethanol solution of the following composition so as to obtain the following amounts of ingredients and to have a thickness after drying of 5 μm (= OC2 application). The fabric was dried, so as to produce an integrated multilayer analytical element.

| | |
|---|---|
| Amylase-labeled anti-canine CRP antibody LA | 19.0 KU/m² |
| | (1.2 mg/m²; molecular weight: 30 KDa) |
| Anti-canine CRP antibody A | 5.4 mg/m² (molecular weight 15 KDa) |
| Polyvinylpyrrolidone | 5.6 g/m² |
| Surfactant | 0.2 g/m² |
| Amylase inhibitor | 2080 KU/m² |

(Calculation of molar ratio of antibodies)

$$\text{Antibody LA } (1.2 \text{mg/m}^2) / \text{molecular weight } (30\,\text{KDa}) = 0.04 \times 10^{-3}\,\text{mmol/m}^2$$

$$\text{Antibody A } (5.4\,\text{mg/m}^2) / \text{molecular weight } (15\,\text{KDa}) = 0.36 \times 10^{-3}\,\text{mmol/ m}^2$$

$$\text{Molar ratio} = 0.36 / 0.04 = 9$$

The aforementioned integrated multilayer analytical element was cut into a chip of 12 mm x 13 mm, and the chip was then placed into a slide frame (described in JP Patent Publication (Kokai) No. 57-63452 (1982) A, so as to produce a dry slide used in CRP analysis according to the present invention. Moreover, the dry analytical elements shown in Table 3 were produced in the same manner as described above with the exception that the types and amounts of the labeled anti-canine CRP antibodies (all of which had a molecular weight of approximately 30 KDa) and the second antibodies (all of which had a molecular weight of approximately 15KDa) were changed as shown in Table 3.

Table 3

| Dry analytical element No. | Labeled antibody/ second antibody | Molar ratio between labeled antibody and second antibody * | Remarks |
|---|---|---|---|
| 101 | LA/A | 9 | The present invention |
| 102 | LB/A | 11 | The present invention |
| 103 | LC/A | 6 | The present invention |
| 104 | LA/C | 10 | The present invention |
| 105 | LA/A | 0.05 | Comparative Example |

(continued)

| Dry analytical element No. | Labeled antibody/ second antibody | Molar ratio between labeled antibody and second antibody * | Remarks |
|---|---|---|---|
| 106 | LA/C | 40 | Comparative Example |
| 107 | LD/A | 10 | Comparative Example |
| * Molar ratio = second antibody/labeled antibody | | | |

Example 4: Sensitivity evaluation test

A diluent with the composition (*) as described below, and a liquid prepared by 21-fold diluting canine serum (CRP concentrations assayed by immunonephelometry: 0, 1.1, 3.0, 7.1 and 10 mg/dL) with the diluent, were added in an amount of 10 $\mu$L to the dry slide used in CRP analysis prepared in the above-described example.

Thereafter, while incubating at 37°C for 5 minutes, the reflection density at 650 nm was measured by Fuji Dri-Chem 5000 (manufactured by Fuji Photofilm Co., Ltd.) every 10 seconds. The reflection density ($\triangle$ODr) per minute was obtained based on the reflection densities from 3 to 5 minutes during the aforementioned measurement. The results are shown in Figure 1.

| | |
|---|---|
| * Composition of diluent | |
| MES * 1 | 5 mg |
| Casein aqueous solution (*2) | 100 mg |
| Sodium azide | 0.2 mg |
| Purified water | 1.0ml |
| * 1 MES: 2-(N-morpholino) ethanesulfonic acid monohydrate | |
| *2 e.g. Trade name: Block Ace | |

From the results shown in Figure 1, in the canine CRP analytical dry slides 101 and 102 of the present invention, $\triangle$ODr (reflection optical density) has changed corresponding to concentration change of canine CRP, up to a canine CRP concentration of 10 mg/dL in canine serum. Thus, a good S/N ratio was obtained. In addition, in the canine CRP analytical dry slides 103 and 104 of the present invention as well, a good S/N ratio was obtained. On the other hand, in the case of the comparative examples 105, 106 and 107, which were out of the scope of the present invention, such a change in $\triangle$ODr (reflection optical density) was poor, corresponding to concentration change of canine CRP, up to a canine CRP concentration of 7.1 mg/dL, and thus, a good S/N ratio could not be obtained.

Example 5: Mutual dilution linearity performance evaluation test

Canine serums having canine CRP concentrations of 0.5 mg/dL and 12 mg/dL, which had been assayed by the immunonephelometry, were mutually diluted with each other to obtain an analyte having a desired CRP concentration. The thus obtained analyte was 21-fold diluted with the same diluent as that used in Example 4, and 10 $\mu$L of the obtained liquid was spotted on the CRP analytical dry slide produced in Example 3.

Thereafter, while incubating it at 37°C for 5 minutes, the reflection density at 650 nm was measured approximately every 10 seconds with Fuji Dri-Chem 700 (manufactured by Fujifilm Holdings Corporation). The reflection density ($\triangle$ODr) per minute was obtained from the reflection density of 3 to 5 minutes. The results are shown in Figure 2.

From the results shown in Figure 2, in the canine CRP analytical dry slide 101 of the present invention, good linearity was observed up to a canine CRP concentration of 12 mg/dL. Moreover, in the canine CRP analytical dry slides 102, 103 and 104 of the present invention as well, good linearity was obtained. On the other hand, in the case of the comparative examples 105 and 106, which were out of the scope of the present invention, such linearity was not observed.

**Claims**

1. A dry analytical element for the measurement of canine CRP, which comprises, on a light-permeable support, an immune reaction layer comprising: a monoclonal antibody fragment prepared by labeling, with an enzyme, a first monoclonal antibody fragment that recognizes canine CRP; and a second monoclonal antibody that recognizes canine CRP, wherein

the first monoclonal antibody and the second monoclonal antibody have a coupling constant of $10^7$ M$^{-1}$ to $10^9$ M$^{-1}$ with respect to canine CRP, the first monoclonal antibody and the second monoclonal antibody are of the IgG1 subclass, and the molar ratio between the first monoclonal antibody and the second monoclonal antibody is 1 to 20.

2. The dry analytical element for the measurement of canine CRP according to claim 1, wherein the molar ratio between the first monoclonal antibody and the second monoclonal antibody is 5 to 15.

3. The dry analytical element for the measurement of canine CRP according to claim 1 or 2, wherein the enzyme is Bacillus subtilis $\alpha$-amylase.

4. The dry analytical element for the measurement of canine CRP according to any one of claims 1 to 3, which further comprises an amylase inhibitor for inhibiting canine amylase.

5. An immunoanalytical reagent or kit for the measurement of canine CRP, which comprises: a monoclonal antibody fragment prepared by labeling, with an enzyme, a first monoclonal antibody fragment that recognizes canine CRP; and a second monoclonal antibody that recognizes canine CRP, wherein

the first monoclonal antibody and the second monoclonal antibody have a coupling constant of $10^7$ M$^{-1}$ to $10^9$ M$^{-1}$ with respect to canine CRP, the first monoclonal antibody and the second monoclonal antibody are of the IgG1 subclass, and the molar ratio between the first monoclonal antibody and the second monoclonal antibody is 1 to 20.

6. The immunoanalytical reagent or kit for the measurement of canine CRP according to claim 5, wherein the molar ratio between the first monoclonal antibody and the second monoclonal antibody is 5 to 15.

7. The immunoanalytical reagent or kit for the measurement of canine CRP according to claim 5 or 6, wherein the enzyme is Bacillus subtilis $\alpha$-amylase.

8. The immunoanalytical reagent or kit for the measurement of canine CRP according to any one of claims 5 to 7, which further comprises an amylase inhibitor for inhibiting canine amylase.

9. A method for measuring canine CRP, wherein the dry analytical element for the measurement of canine CRP of any one of claims 1 to 4 or the immunoanalytical reagent or kit for the measurement of canine CRP of any one of claims 5 to 8 is used.

## Patentansprüche

1. Trockenes Analyseelement zur Messung von kaninem CRP, umfassend, auf einem lichtdurchlässigen Träger, eine Immunreaktionsschicht, die umfasst: ein monoklonales Antikörperfragment, das durch Markierung eines ersten monoklonalen Antikörperfragments, das kanines CRP erkennt, mit einem Enzym, hergestellt ist; und einen zweiten monoklonalen Antikörper, der kanines CRP erkennt, wobei

der erste monoklonale Antikörper und der zweite monoklonale Antikörper eine Kopplungskonstante von $10^7$ M$^{-1}$ bis $10^9$ M$^{-1}$ bezüglich kaninem CRP aufweisen, der erste monoklonale Antikörper und der zweite monoklonale Antikörper zu der IgG1-Unterklasse gehören und das Molverhältnis zwischen dem ersten monoklonalen Antikörper und dem zweiten monoklonalen Antikörper 1 bis 20 beträgt.

2. Trockenes Analyseelement zur Messung von kaninem CRP gemäß Anspruch 1, wobei das Molverhältnis zwischen dem ersten monoklonalen Antikörper und dem zweiten monoklonalen Antikörper 5 bis 15 beträgt.

3. Trockenes Analyseelement zur Messung von kaninem CRP gemäß Anspruch 1 oder 2, wobei das Enzym Bacillus sutilis $\alpha$-Amylase ist.

4. Trockenes Analyseelement zur Messung von kaninem CRP gemäß einem der Ansprüche 1 bis 3, das ferner einen Amylase-Inhibitor zur Hemmung kaniner Amylase umfasst.

5. Immunoanalytisches Reagens oder Kit zur Messung von kaninem CRP, umfassend: ein monoklonales Antikörperfragment, das durch Markierung eines ersten monoklonalen Antikörperfragments, das kanines CRP erkennt, mit einem Enzym, hergestellt ist; und einen zweiten monoklonalen Antikörper, der kanines CRP erkennt, wobei der erste monoklonale Antikörper und der zweite monoklonale Antikörper eine Kopplungskonstante von $10^7$ M$^{-1}$ bis $10^9$ M$^{-1}$ bezüglich kaninem CRP aufweisen, der erste monoklonale Antikörper und der zweite monoklonale Antikörper zu der IgG1-Unterklasse gehören und das Molverhältnis zwischen dem ersten monoklonalen Antikörper und dem zweiten monoklonalen Antikörper 1 bis 20 beträgt.

**6.** Immunoanalytisches Reagens oder Kit zur Messung von kaninem CRP gemäß Anspruch 5, wobei das Molverhältnis zwischen dem ersten monoklonalen Antikörper und dem zweiten monoklonalen Antikörper 5 bis 15 beträgt.

**7.** Immunoanalytisches Reagens oder Kit zur Messung von kaninem CRP gemäß Anspruch 5 oder 6, wobei das Enzym Bacillus sutilis α-Amylase ist.

**8.** Immunoanalytisches Reagens oder Kit zur Messung von kaninem CRP gemäß einem der Ansprüche 5 bis 7, das ferner einen Amylase-Inhibitor zur Hemmung kaniner Amylase umfasst.

**9.** Verfahren zur Messung von kaninem CRP, wobei das trockene Analyseelement zur Messung von kaninem CRP gemäß einem der Ansprüche 1 bis 4 oder das immunoanalytische Reagens oder Kit zur Messung von kaninem CRP gemäß einem der Ansprüche 5 bis 8 eingesetzt wird.

## Revendications

**1.** Elément analytique sec pour la mesure de la CRP canine, qui comprend, sur un support perméable à la lumière, une couche de réaction immunitaire comprenant : un fragment d'anticorps monoclonal préparé par marquage, avec une enzyme, d'un fragment d'un premier anticorps monoclonal qui reconnait la CRP canine ; et un second anticorps monoclonal qui reconnait la CRP canine, où
le premier anticorps monoclonal et le second anticorps monoclonal ont une constante de couplage de $10^7$ M$^{-1}$ à $10^9$ M$^{-1}$ à l'égard de la CRP canine, le premier anticorps monoclonal et le second anticorps monoclonal sont de la sous-classe IgG1, et le rapport molaire entre le premier anticorps monoclonal et le second anticorps monoclonal est 1 à 20.

**2.** Elément analytique sec pour la mesure de la CRP canine selon la revendication 1 où le rapport molaire entre le premier anticorps monoclonal et le second anticorps monoclonal est 5 à 15.

**3.** Elément analytique sec pour la mesure de la CRP canine selon la revendication 1 ou 2, où l'enzyme est l'α-amylase de Bacillus subtilis.

**4.** Elément analytique sec pour la mesure de la CRP canine selon l'une quelconque des revendications 1 à 3, qui comprend en outre un inhibiteur d'amylase pour inhiber l'amylase canine.

**5.** Réactif ou kit immunoanalytique pour la mesure de la CRP canine qui comprend : un fragment d'anticorps monoclonal préparé par marquage, avec une enzyme, d'un fragment d'un premier anticorps monoclonal qui reconnait la CRP canine ; et un second anticorps monoclonal qui reconnait la CRP canine, où
le premier anticorps monoclonal et le second anticorps monoclonal ont une constante de couplage de $10^7$ M$^{-1}$ à $10^9$ M$^{-1}$ à l'égard de la CRP canine, le premier anticorps monoclonal et le second anticorps monoclonal sont de la sous-classe IgG1, et le rapport molaire entre le premier anticorps monoclonal et le second anticorps monoclonal est 1 à 20.

**6.** Réactif ou kit immunoanalytique pour la mesure de la CRP canine selon la revendication 5, où le rapport molaire entre le premier anticorps monoclonal et le second anticorps monoclonal est 5 à 15.

**7.** Réactif ou kit immunoanalytique pour la mesure de la CRP canine selon la revendication 5 ou 6, où l'enzyme est l'α-amylase de Bacillus subtilis.

**8.** Réactif ou kit immunoanalytique pour la mesure de la CRP canine selon l'une quelconque des revendications 5 à 7 qui comprend en outre un inhibiteur d'amylase pour inhiber l'amylase canine.

9. Procédé pour mesurer la CRP canine où l'élément analytique sec pour la mesure de la CRP canine selon l'une quelconque des revendications 1 à 4 ou le réactif ou kit immunoanalytique pour la mesure de la CRP canine selon l'une quelconque des revendications 5 à 8 est utilisé.

Figure 1

ΔODr/min to CRP

Figure 2

Dilution linearity

Present Invention 101
△ Comparative Example 105
○ Comparative Example 106

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 62210984 A
- JP 3151080 B
- JP 62155299 A
- JP 61243363 A
- JP 60108756 A
- JP 60171461 A
- JP 60171460 A
- JP 49053888 A
- US 3992158 A
- JP 51040191 A
- US 4042335 A
- JP 55164356 A
- US 4292272 A
- JP 61004959 A
- EP 0166365 A
- JP 55090859 A
- US 4258001 A
- JP 58070163 A
- US 4486537 A
- JP 62116258 A
- JP 62138756 A
- EP 0226465 A
- JP 62138757 A
- JP 62138758 A
- JP 57066359 A
- JP 60222770 A
- EP 0162301 A
- JP 63219397 A
- DE 3717913 A
- JP 63112999 A
- JP 62182652 A
- JP 53021677 B
- JP 54101398 A
- US 4132528 A
- JP 61292063 A
- JP 3295466 A
- EP 0451848 A
- JP 4128655 A
- JP 42765551 A
- JP 49050991 A
- US 3886045 A
- JP 53026188 A
- US 4089747 A
- JP 58045557 A
- JP 59193352 A
- EP 0122641 A
- JP 60224677 A
- US 4665023 A
- JP 57028331 B
- US 4169751 A
- JP 56142454 U
- US 4387990 A
- JP 57063452 A
- JP 58032350 U
- JP 1983 U
- JP 58501144 A
- WO 8300391 A
- JP 60125543 A
- JP 60220862 A
- JP 61294367 A

### Non-patent literature cited in the description

- *Vet. clin. Pathol.,* 2003, vol. 32, 81-87
- *Vet clin. Pathol.,* 2003, vol. 32, 81-87
- **C. A. WILLIAMS ; M. W. CHASE.** Method in Immunology and Immunochemistry. Academic Press, 1967, vol. 1
- Koso Meneki Sokutei Ho (Enzyme Immunoassay. Igaku-Shoin, Ltd, 1978
- Kagaku Binran Kiso Hen. Chemical Society of Japan, Tokyo, Mazuren Co., Ltd, 1966, 1312-1320
- Data for Biochemical Research. Oxford at the Clarendon Press, 1969, 476-508
- *Biochemistry,* 1966, vol. 5, 467-477
- *Analytical Biochemistry,* 1980, vol. 104, 300-310
- **BUNJI MARUO ; NOBUO TAMIYA.** Koso Handbook. Asakura Publishing Co., Ltd, 1982
- **NOBUMASA IMURA et al.** Seikagaku Handbook. Maruzen Co., Ltd, 1984
- *Ann. Clin. Biochem.,* 1964, vol. 6, 24
- *J. Clin. Pathol.,* 1969, vol. 22, 246